Europäisches Patentamt

European Patent Office

Office européen des brevets

Veröffentlichungsnummer: **0 274 607**

**A1**

# EUROPÄISCHE PATENTANMELDUNG

Anmeldenummer: **87116768.0**

Int. Cl.⁴ **A61B 17/16**

Anmeldetag: **13.11.87**

Priorität: **03.12.86 DE 3641296**

Veröffentlichungstag der Anmeldung:
**20.07.88 Patentblatt 88/29**

Benannte Vertragsstaaten:
**AT BE CH DE ES FR GB GR IT LI LU NL SE**

Anmelder: **List, Heinz-Jürgen**
**Lindenstrasse 56**
**D-7990 Friedrichshafen 1(DE)**

Erfinder: **Richter, Ulrich**
**Ludolfinger Strasse 14**
**D-8500 Nürnberg 50(DE)**

Vertreter: **Göbel, Matthias, Dipl.-Ing.**
**Pruppacher Hauptstrasse 5-7**
**D-8501 Pyrbaum-Pruppach(DE)**

## Medizinische Bohrmaschine.

Bei einer medizinischen Bohrmaschine mit einem im Bohrmaschinengehäuse (1) fixierten Elektromotor (5), der über mindestens eine Getriebeanordnung ein Spannelement (9) für Bohrwerkzeuge antreibt, ist zur stoß-und erschütterungsfreien Lagerung des Elektromotors (5) im Bohrmaschinengehäuse (1) vorgesehen, daß das Gehäuse (5') des Elektromotors (5) durch Ringkörper (10) aus einem flexiblen oder federnd elastischen Werkstoff pressend umfaßt und durch mindestens einen zwischen Motorgehäuse (5') und Bohrmaschinengehäuse (1) eingespannten federnd elastischen Stützkörper (12) im Bohrmaschinengehäuse (1) radial und axial elastisch nachgiebig fixiert ist.

Fig. 1

## Medizinische Bohrmaschine

Die Erfindung betrifft eine medizinische Bohrmaschine mit einem im Bohrmaschinengehäuse fixierten Elektromotor, der über mindestens eine Getriebeanordnung ein Spannelement für Bohrwerkzeuge antreibt.

Es ist bei technischen und medizinischen Bohrmaschinen bekannt, den Elektromotor im Bohrmaschinengehäuse starr festzulegen. Diese Halterung des Elektromotors ist umständlich und kompliziert und führt dazu, daß insbesondere bei Drehmomentänderungen Stöße am Bohrwerkzeug wirksam werden, die sich bei medizinischen Bohrmaschinen wegen des empfindlichen Bohrgutes ungünstig auswirken. Außerdem werden vom Elektromotor Erschütterungen und Vibrationen auf das Bohrmaschinengehäuse und weiter auf das Bohrwerkzeug gelegt, die die Handhabung von medizinischen Bohrmaschinen erschweren.

Es ist Aufgabe der Erfindung, Maßnahmen zur stoß- und erschütterungsfreien Lagerung des Elektromotors in Gehäusen von medizinischen Bohrmaschinen einfach zu schaffen.

Der Erfindung gemäß ist diese Aufgabe dadurch gelöst, daß das Gehäuse des Elektromotors durch Ringkörper aus einem flexiblen oder federnd elastischen Werkstoff pressend umfaßt und durch einen zwischen Motorgehäuse und Bohrmaschinengehäuse eingespannten federnd elastischen Stützkörper im Bohrmaschinengehäuse radial und axial elastisch nachgiebig fixiert ist. Zweckmäßig finden zwei Ringkörper Anwendung, die an bohrmaschinengehäusefesten Leisten abstützbar sind. Auf diese Weise fehlen starre Verbindungen zwischen Elektromotor und Bohrmaschinengehäuse und der Elektromotor ist freischwingend im Bohrmaschinengehäuse mit Drehmomentabstützung gehalten, wodurch auch das Bohrwerkzeug frei von Erschütterungen, Vibrationen usw. bleibt. Gleichzeitig bringen Ringkörper und Stützkörper Reaktionskräfte gegen Verdrehungen des Motorgehäuses auf, wodurch ein weicher Drehmomenteinsatz gewährleistet ist und der Elektromotor mit seinem Gehäuse jeweils selbsttätig in seine Ausgangsstellung zurückdreht. Durch entsprechende Bemessung der Ringkörper und des Stützkörpers oder deren Anzahl, kann eine exakte Anpassung an die jeweils notwendigen Haltekräfte erreicht werden.

In Ausgestaltung der Bohrmaschine ist noch vorgesehen, daß die Ringkörper in Nuten des Bohrmaschinengehäuses eingreifen und in diesen durch die Begrenzungsflächen derselben abgestützt sind.

In weiterer Ausgestaltung der Bohrmaschine ist als Stützkörper ein Zapfen vorgesehen, der mit seinem einen Ende an einer Stirnfläche des Motorgehäuses und mit seinem anderen Ende an einem bohrmaschinengehäusefesten Ansatz fest angreift. Die Festlegung des Zapfens kann durch Klemmittel, Klebung oder Vulkanisation erfolgen. Schließlich kann die Verbindung von Zapfen und Ansatz auch durch einen an dem dem Motorgehäuse abgewandten Ende des Zapfens angeordneten Gewindeansatz erreicht sein, der mit einem bohrmaschinengehäusefesten Ansatz verschraubt ist. Maßgeblich ist, daß zwischen dem Motorgehäuse und dem Bohrmaschinengehäuse federnd elastische Streckenabschnitte vorhanden sind, die die Übertragung von Stößen auf das Bohrmaschinengehäuse verhindern. Der Zapfen kann vorteilhaft aus einem Gummiwerkstoff oder einem federnd elastischen Kunststoff gebildet sein.

Die Erfindung ist anhand eines Ausführungsbeispiels in der Zeichnung erläutert. Es bedeuten:

Fig. 1 ein Teilstück einer medizinischen Bohrmaschine, teilweise hälftig geöffnet,

Fig. 2 einen Elektromotor, perspektivisch und

Fig. 3 eine Befestigungsstelle von Elektromotor und Bohrmaschinengehäuse, vergrößert.

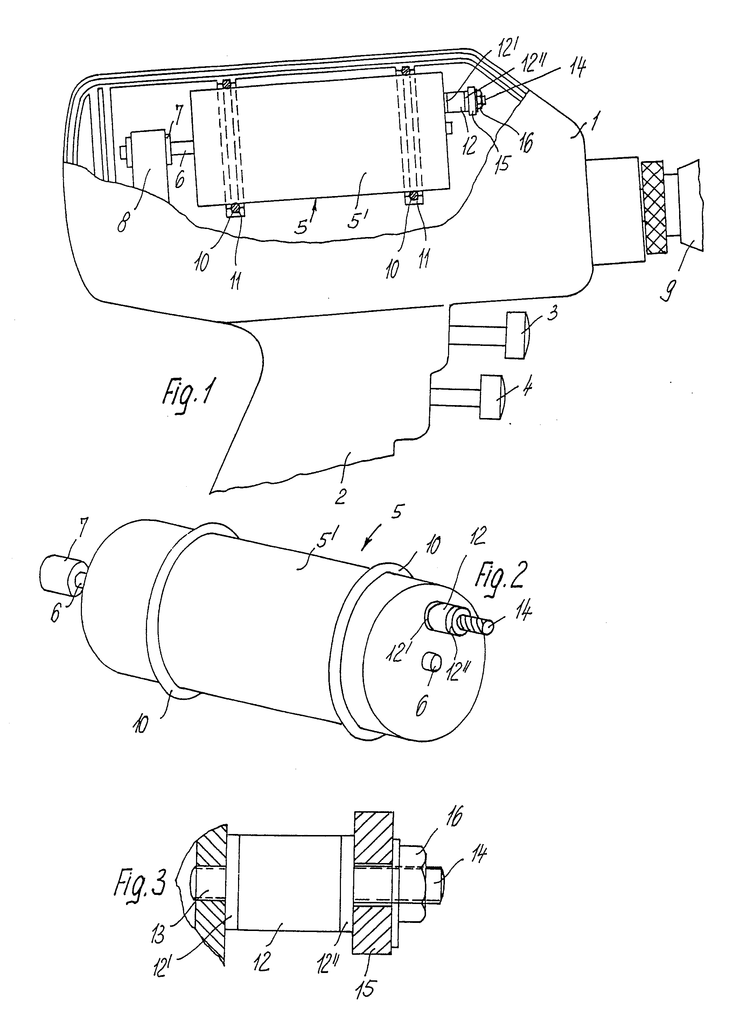

In den Fig. ist mit 1 ein Bohrmaschinengehäuse bezeichnet, das an seiner Unterseite einen Griff 2 aufweist, der Betätigungsknöpfe 3, 4 für einen im Bohrmaschinengehäuse 1 angeordneten Elektromotor 5 trägt. Mittels der Betätigungsknöpfe 3, 4 ist der Elektromotor 4 an eine Stromquelle (nicht gezeigt) anlegbar und auf Links- oder Rechtslauf bzw. Stop stellbar. Der Elektromotor 5 treibt über eine Motorwelle 6 ein Ritzel 7 an, das weiter über ein Band 8 eine Abtriebswelle (nicht gezeigt) für ein Spannfutter 9 dreht. Der Elektromotor 5 ist im Gehäuse 1 freischwingend und sowohl in axialer als auch radialer Richtung elastisch nachgiebig gehalten. Hierzu trägt das Gehäuse 5' des Elektromotors 5 außen zwei Ringkörper 10 aus einem federnd elastischen Werkstoff, z. B. einem geeigneten Kunststoff, die beim Ausführungsbeispiel in Nuten 11 des Gehäuses 1 eintauchen und sich an den Begrenzungsflächen der Nuten 11 abstützen. An einer Stirnseite 5" des Gehäuses 5' des Elektromotors 5 greift weiter ein Zapfen 12 aus federnd elastischem Werkstoff fest an, der hierzu mittels einer Platte 12' und eines Gewindezapfens 13 (Fig. 3) im Gehäuse 1 verschraubt ist. Der Zapfen 12 trägt an seinem dem Elektromotor 5 abgewandten Ende eine Platte 12" mit Gewindeansatz 14, der einen gehäusefesten Ansatz 15 durchgreift und mittels einer Mutter 16 fixiert ist. Durch Verformungsarbeit von Zapfen 12 und Ringkörper 10 werden bei Belastungen des Elektromotors 5 Reaktionskräfte an diesen wirksam, die eine nachgiebige Rückstellung des Elektromotors 5 in seine Ausgangslage bewirken. Die Platten 12', 12" können mit den Zapfen 12 durch Klebung oder Vulkanisation fest verbunden sein. Es besteht auch die Möglichkeit Zapfen 12 und Platten 12', 12" mechanisch, z. B. mittels Schraubansätze miteinander zu verbinden. Schließlich ist auch denkbar, mehr als zwei Ringkörper 10 für die Abstützung des Elektromotors im Gehäuse 1 in Anwendung zu bringen.

**Ansprüche**

1. Medizinische Bohrmaschine mit einem im Bohrmaschinengehäuse fixierten Elektromotor, der über mindestens eine Getriebeanordnung ein Spannelement für Bohrwerkzeuge antreibt, dadurch gekennzeichnet, daß das Gehäuse (5') des Elektromotors (5) durch Ringkörper (10) aus einem flexiblen oder federnd elastischen Werkstoff pressend umfaßt und durch mindestens einen zwischen Motorgehäuse (5') und Bohrmaschinengehäuse (1) eingespannten federnd elastischen Stützkörper (12) im Bohrmaschinengehäuse (1) radial und axial elastisch nachgiebig fixiert ist.

2. Bohrmaschine nach Anspruch 1, dadurch gekennzeichnet, daß zwei Ringkörper (10) das Motorgehäuse (5') umfassen und daß die Ringkörper (10) an bohrmaschinengehäusefeste, Leisten abstützbar sind.

3. Bohrmaschine nach Anspruch 1, dadurch gekehnzeichnet, daß die Ringkörper (10) und der Stützkörper (12) Reaktionskräfte gegen Verdrehung auf das Motorgehäuse (5') ausüben.

4. Bohrmaschine nach Anspruch 1, 2 und 3, dadurch gekennzeichnet, daß die Ringkörper (10) in Nuten (11) des Bohrmaschinengehäuses (1) eingreifen und durch die Begrenzungsflächen der Nuten gestützt sind.

5. Bohrmaschine nach Anspruch 1, dadurch gekennzeichnet, daß als Stützkörper ein Zapfen (12) dient, der mit seinem einen Ende an einer Stirnfläche (5") des Motorgehäuses (5') und mit seinem anderen Ende an einem bohrmaschinengehäusefesten Ansatz (15) fest angeordnet ist.

6. Bohrmaschine nach Anspruch 5, dadurch gekennzeichnet, daß der Zapfen (12) vermittels Klemmittel, durch Klebung oder Vulkanisation mit dem Motorgehäuse fest verbunden ist.

7. Bohrmaschine nach Anspruch 5, dadurch gekennzeichnet, daß der Zapfen (12) durch einen am Motorgehäuse (5') abgewandten Ende angeordneten Gewindeansatz (14) mit einem bohrmaschinengehäusefesten Ansatz (15) verschraubt ist.

8. Bohrmaschine nach Anspruch 7, dadurch gekennzeichnet, daß der Zapfen (12) aus einem Gummiwerkstoff oder einem federnd elastischen Kunststoff gebildet ist.

0 274 607

Fig.1

Fig.2

Fig.3

# EUROPÄISCHER RECHERCHENBERICHT

**Europäisches Patentamt**

Nummer der Anmeldung

EP 87 11 6768

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-1 381 387 (STRAD INSTRUMENTATION LTD.) * Seite 2, Zeilen 4-57; Ansprüche 5-8; Figur 1 * --- | 1,8 | A 61 B 17/16 |
| A | US-A-4 338 052 (LOCKETT) * Spalte 3, Zeilen 8-20; Ansprüche 1-3; Figur 1 * --- | 1,3,8 | |
| A | GB-A-1 337 944 (BOSCH) * Seite 2, Zeilen 61-117; Figur 1 * ----- | 1,8 | |

| RECHERCHIERTE SACHGEBIETE (Int. Cl.4) |
|---|
| A 61 B 17/00 B 23 B 45/00 B 23 Q 11/00 |

Der vorliegende Recherchenbericht wurde für alle Patentansprüche erstellt

| Recherchenort | Abschlußdatum der Recherche | Prüfer |
|---|---|---|
| BERLIN | 01-04-1988 | MONNE E.M.B. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)